(19) Europäisches Patentamt
European Patent Office
Office européen des brevets

(11) EP 3 164 382 B1

(12) FASCICULE DE BREVET EUROPEEN

(45) Date de publication et mention de la délivrance du brevet:
**16.01.2019 Bulletin 2019/03**

(21) Numéro de dépôt: **15736579.2**

(22) Date de dépôt: **29.06.2015**

(51) Int Cl.:
***C07C 319/02*** (2006.01) ***C07C 319/24*** (2006.01)
***C07C 321/14*** (2006.01) ***C07C 321/04*** (2006.01)
***C01B 32/75*** (2017.01)

(86) Numéro de dépôt international:
**PCT/FR2015/051761**

(87) Numéro de publication internationale:
**WO 2016/001554 (07.01.2016 Gazette 2016/01)**

# (54) PROCÉDÉ DE PRÉPARATION DE DISULFURE DE DIMÉTHYLE

VERFAHREN ZUR HERSTELLUNG VON DIMETHYLDISULFID

METHOD FOR PREPARING DIMETHYL DISULPHIDE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **04.07.2014 FR 1456440**

(43) Date de publication de la demande:
**10.05.2017 Bulletin 2017/19**

(73) Titulaire: **Arkema France**
**92700 Colombes (FR)**

(72) Inventeurs:
- **FREMY, Georges**
**64390 Sauveterre de Bearn (FR)**
- **BARRE, Patrice**
**64140 Lons (FR)**
- **RAYMOND, Jean-Michel**
**40300 Cauneille (FR)**

(56) Documents cités:
**EP-A1- 0 446 109** **US-A- 3 755 461**
**US-A- 3 880 933**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente invention concerne un procédé de préparation de disulfure de diméthyle (dont l'acronyme utilisé par la suite est DMDS) à partir d'une charge hydrocarbonée, de sulfure d'hydrogène et de soufre.

Les disulfures, et notamment le disulfure de diméthyle, présentent un grand intérêt industriel et sont aujourd'hui très largement utilisés dans l'industrie, par exemple et de manière non limitative, en tant qu'additif de sulfuration de catalyseurs, notamment d'hydrotraitement de coupes pétrolières, ou encore comme agent de fumigation des sols dans l'agriculture.

Aujourd'hui le DMDS est couramment produit en gros tonnage industriellement par action du soufre sur le méthylmercaptan, comme décrit par exemple dans les documents EP337837 et EP446109.

Le méthylmercaptan peut être lui-même produit à partir de méthanol ($CH_3OH$) et de sulfure d'hydrogène ($H_2S$) selon la réaction (1) suivante :

$$CH_3OH + H_2S \rightarrow CH_3SH + H_2O \qquad (1)$$

Cependant cette voie de synthèse présente plusieurs inconvénients, parmi lesquels on peut citer celui d'utiliser du méthanol, ce qui nécessite une étape supplémentaire, le méthanol étant préparé à partir de charges hydrocarbonées, l'inconvénient de conduire à des produits secondaires, notamment de type diméthyléther ($CH_3OCH_3$), sulfure de diméthyle ($CH_3SCH_3$), et des produits de craquage (tels que par exemple le monoxyde de carbone et le dioxyde de carbone) et de l'eau, pour ne citer que quelques-uns des inconvénients. En outre, la présence de tels produits secondaires engendre un nombre important d'étapes de purification du méthylmercaptan, au détriment d'une productivité et d'une sélectivité élevées et donc d'un rendement optimal.

Cette voie de synthèse, ainsi que certaines de ses améliorations, sont par exemple décrite dans les documents WO2004/096760, WO2006/015668, WO2007/028708, WO2008/118925 et WO2013/092129.

D'autres procédés de synthèse permettent de s'affranchir de l'utilisation de méthanol, et parmi ceux-ci, on peut citer la préparation de méthylmercaptan à partir de monoxyde de carbone (CO) selon la réaction (2) suivante :

$$CO + 2H_2 + H_2S \rightarrow CH_3SH + H_2O \qquad (2)$$

Toutefois la mise en oeuvre de monoxyde de carbone (CO) n'est pas sans inconvénient, le CO provenant essentiellement de gaz de synthèse qui est un mélange $CO/H_2$ et qui nécessite par conséquent :

- une étape supplémentaire de reformage à la vapeur d'une source hydrocarbonée en vue d'obtenir un gaz de synthèse,
- de disposer d'un gaz de synthèse ayant les proportions adéquates en monoxyde de carbone (CO) et hydrogène ($H_2$) sans quoi il est nécessaire d'ajuster le ratio $CO/H_2$ à l'aide du procédé dit de « gaz à l'eau » défini comme suit dans la réaction (A) :

$$CO + H_2O \rightarrow CO_2 + H_2 \qquad (A)$$

En outre, les procédés suivant la réaction (2) ci-dessus présentent l'inconvénient de générer des produits secondaires, tels que le dioxyde de carbone ($CO_2$), du méthane ($CH_4$), du sulfure de diméthyle ($CH_3SCH_3$) et de l'eau ($H_2O$). Ces procédés sont par exemple décrits dans les documents US2007213564, US2008293974, US2010094059, et US2010286448.

D'autres procédés encore sont décrits dans la littérature et combinent différentes réactions telles que :

- formation de $CS_2$ et d'$H_2S$ à partir de méthane et de soufre, selon la réaction (3) :

$$CH_4 + 4\ S \rightarrow CS_2 + 2\ H_2S \qquad (3)$$

- hydrogénation du $CS_2$, avec l'hydrogène formé ci-dessus, selon la réaction (4) :

$$CS_2 + 3\ H_2 \rightarrow CH_3SH + H_2S \qquad (4)$$

Ces procédés combinent évidemment les inconvénients décrits pour les réactions (1) et (2) avec la difficulté supplémentaire de nécessiter une source supplémentaire d'hydrogène pour effectuer la réaction (4).

Une autre méthode encore est divulguée dans le document WO2010/046607 et consiste en l'hydrogénation de composés soufrés porteurs d'une insaturation de type C=S, et plus particulièrement l'hydrogénation du disulfure de carbone ($CS_2$), en méthylmercaptan ($CH_3SH$) selon la réaction (4) ci-dessus. Le brevet US 3,880,933 divulgue également un procédé de conversion de disulfure de carbone en méthylmercaptan en présence d'hydrogène, ledit disulfure de carbone pouvant être obtenu à partir de méthane et de sulfure d'hydrogène.

Cependant le procédé mis en oeuvre dans ce document fait appel à du disulfure de carbone ($CS_2$), qui est un produit, dangereux, toxique et utilisable sur un plan industriel moyennant la mise en place de moyens drastiques de sécurité ; et toutes les entreprises et usines, ne souhaitent pas ou ne peuvent pas développer une installation répondant aux normes de sécurité nécessaires à la manutention de disulfure de carbone.

La demande internationale WO2001/96290 propose un procédé de synthèse de méthylmercaptan directement à partir de méthane ($CH_4$) et d'$H_2S$ avec coproduction d'hydrogène. Cette réaction directe entre le méthane et l'$H_2S$ se fait à l'aide d'un plasma pulsé avec décharge en couronne. Cette demande ne décrit aucun exemple de synthèse, et il ne semble pas possible d'envisager la mise à l'échelon industriel de ce procédé de synthèse de méthylmercaptan. De plus ce procédé nécessite la synthèse de l'$H_2S$ si celui-ci n'est pas disponible.

Il existe par conséquent aujourd'hui un besoin pour un procédé de synthèse de DMDS par action du soufre sur le méthylmercaptan ne présentant pas les inconvénients rencontrés dans les procédés connus, c'est-à-dire plus respectueux de l'environnement moins éco-toxique, mais aussi plus sûr, tout en conservant des rendements et sélectivités élevés, voire des rendements et sélectivités améliorés par rapport aux procédés connus, tout en étant un procédé opéré de la manière la plus économique possible.

Il a maintenant été découvert qu'il est possible de s'affranchir des inconvénients précités, en totalité ou tout au moins en partie, grâce au procédé de préparation de disulfure de diméthyle (DMDS) selon la présente invention qui est détaillée dans la description qui suit. En particulier, le procédé de la présente invention permet de résoudre un grand nombre des inconvénients des procédés basés sur les réactions (1) et (2) décrites *supra.*

Ainsi, et selon un premier aspect, la présente invention a pour objet un procédé de préparation de disulfure de diméthyle, en batch ou en continu, de préférence en continu, ledit procédé comprenant au moins les étapes suivantes :

a) réaction d'au moins une charge hydrocarbonée en présence de sulfure d'hydrogène ($H_2S$) et optionnellement de soufre (S) pour former du disulfure de carbone ($CS_2$) et de l'hydrogène ($H_2$),
b) réaction d'hydrogénation dudit disulfure de carbone ($CS_2$) en présence dudit hydrogène ($H_2$) obtenus à l'étape a), pour former du méthylmercaptan ($CH_3SH$), du sulfure d'hydrogène($H_2S$) et éventuellement de l'hydrogène ($H_2$),
c) éventuellement, mais de préférence, recyclage dudit sulfure d'hydrogène($H_2S$) formé à l'étape b) vers l'étape a),
d) réaction du méthylmercaptan formé à l'étape b) avec du soufre pour former du disulfure de diméthyle et du sulfure d'hydrogène,
e) éventuellement recyclage dans l'étape a) du sulfure d'hydrogène formé à l'étape d), et
f) récupération du disulfure de diméthyle.

Ce procédé présente le très grand avantage de consommer le sulfure d'hydrogène ($H_2S$) qui est produit au cours de la réaction, et dans certains cas même de manière stoechiométrique comme indiqué plus loin, c'est-à-dire que tout le sulfure d'hydrogène consommé dans le procédé de l'invention est produit par ledit procédé. Ainsi, le procédé de l'invention permet d'éviter toute addition, voire dans certains cas toute élimination de sulfure d'hydrogène ($H_2S$) en excès ou encore permet d'éviter la synthèse additionnelle de sulfure d'hydrogène ($H_2S$), comme il est parfois requis avec les procédés connus de l'art antérieur.

En outre, le procédé de la présente invention est un procédé simple à mettre en oeuvre, de faible éco-toxicité et économique. Le procédé de l'invention permet également d'obtenir un haut rendement et une haute sélectivité en DMDS. Dans la présente description et sauf mention contraire, les pourcentages mentionnés sont des pourcentages en poids.

Le procédé selon l'invention est un procédé en trois étapes réactionnelles consécutives (étapes a), b) et d) ci-dessus), sans qu'il soit nécessaire d'effectuer de purification des intermédiaires obtenus à chaque étape. De manière schématique, la première étape du procédé (étape a) est une réaction, effectuée de préférence à haute température, entre une charge hydrocarbonée (illustré ici : le méthane) et du sulfure d'hydrogène ($H_2S$) selon la réaction (5) :

$$CH_4 + 2\ H_2S \rightarrow CS_2 + 4\ H_2 \qquad (5)$$

Dans la deuxième étape (étape b)), le disulfure de carbone formé à l'étape a) est soumis à hydrogénation catalytique avec l'hydrogène également formé à l'étape a), selon la réaction (6) :

$$CS_2 + 4\ H_2 \rightarrow CH_3SH + H_2S + H_2 \qquad (6)$$

Cette succession des deux étapes réactionnelles permet de constater que le nombre de moles de méthylmercaptan formé est identique au nombre de moles de méthane consommé, et que le l'étape a) (réaction 5) nécessite deux fois plus de moles de sulfure d'hydrogène qu'il n'en est formé lors de l'étape b) (réaction 6).

Dans un mode de réalisation tout particulièrement avantageux de la présente invention, le sulfure d'hydrogène formé dans l'étape b) est recyclé dans l'étape a) selon l'étape c). Dans ce mode de réalisation, il est observé que la totalité du sulfure d'hydrogène formé peut ainsi être réutilisé dans l'étape a), ceci permettant d'éviter le stockage dudit sulfure d'hydrogène formé.

Dans un autre mode de réalisation, l'hydrogène sulfuré (ou sulfure d'hydrogène) formé à l'issu de l'étape b) peut ne pas être recyclé dans l'étape a) et récupéré pour être utilisé ultérieurement, par exemple être conduit dans l'étape e) de recyclage du sulfure d'hydrogène formé après la réaction du soufre sur le méthylmercaptan.

Comme indiqué plus haut, la quantité d'hydrogène sulfuré produit à l'étape b) (réaction (6) ci-dessus) n'est pas en quantité molaire suffisante pour la conduite de la réaction (5) de l'étape a) et une quantité supplémentaire de sulfure d'hydrogène doit être apportée pour conduire l'étape a). Cette quantité supplémentaire peut par exemple provenir du sulfure d'hydrogène formé après la réaction du soufre sur le méthylmercaptan et qui peut être recyclé selon l'étape e).

En variante, il peut être également envisagé de synthétiser la quantité de sulfure d'hydrogène manquante, notamment à partir de l'hydrogène formé à l'étape b) mis en réaction avec du soufre selon le procédé décrit par exemple dans le document WO2004/022482, selon la réaction (B) suivante :

$$S + H_2 \rightarrow H_2S \qquad (B)$$

Le méthylmercaptan formé à l'étape b), et après recyclage éventuel du sulfure d'hydrogène($H_2S$) à l'étape c), est ensuite mis à réagir avec du soufre selon les méthodes classiques connues de l'homme du métier, par exemple comme décrit dans EP0976726, selon le schéma réactionnel (8) suivant :

$$2\ CH_3SH + S \rightarrow CH_3SSCH_3 + H_2S \qquad (8)$$

La réaction du méthylmercaptan avec du soufre pour former du disulfure de diméthyle est tout à fait intéressante, y compris sur le plan économique, en raison de la formation simultanée de sulfure d'hydrogène qui peut tout à fait avantageusement être recyclé dans l'étape a) du procédé de l'invention de synthèse de méthylmercaptan.

Selon une variante du procédé de l'invention, le soufre peut être introduit dès la première étape (étape a)). La réaction équilibrée peut alors s'écrire selon le schéma (9) ci-dessous :

$$CH_4 + S + H_2S \rightarrow CS_2 + 3\ H_2 \qquad (9)$$

et l'étape b) du procédé peut alors être illustrée par le schéma réactionnel (4) :

$$CS_2 + 3\ H_2 \rightarrow CH_3SH + H_2S \qquad (4)$$

Dans ce cas, la totalité de l'hydrogène sulfuré produit à l'étape b) selon la réaction du schéma (4) peut avantageusement être recyclé totalement (stoechiométrie respectée) à l'étape a) (dans le schéma (9)), ceci évitant une synthèse supplémentaire de sulfure d'hydrogène avec des équipements supplémentaires.

Ainsi, et selon que l'étape a) ne comprenne pas l'addition de soufre ou comprenne l'addition de soufre, et considérant le bilan-matières global, le procédé selon l'invention présente le très grand avantage de produire une mole de méthylmercaptan par mole de méthane consommé. Ces modes de réalisation de la présente invention peuvent être schématisés comme illustré respectivement par les schémas réactionnels ($\alpha$) et ($\beta$) suivants :

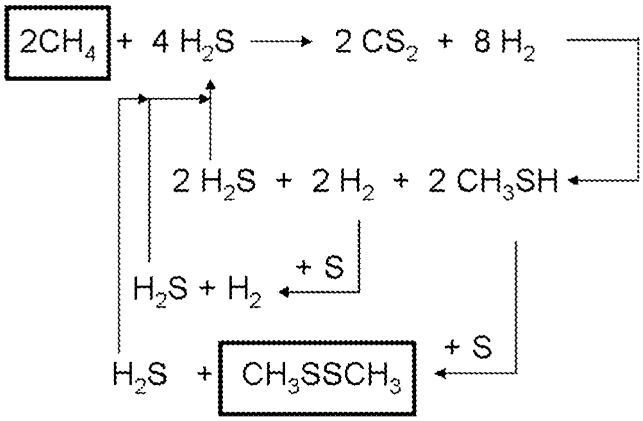

(α)

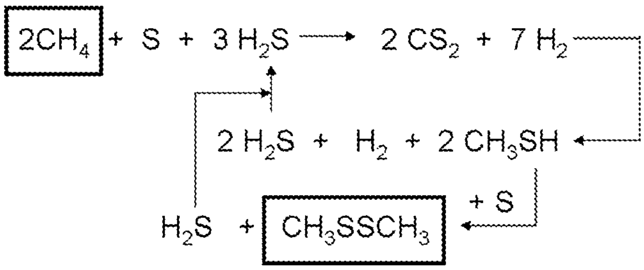

(β)

étant entendu que, dans ces schémas les recyclages du sulfure d'hydrogène sont optionnels, et que la synthèse de sulfure d'hydrogène à partir d'hydrogène et de soufre est également optionnelle.

Toutes les réactions ci-dessus font apparaître le méthane ($CH_4$) comme charge hydrocarbonée de départ, mais le procédé de l'invention peut être réalisé de façon similaire à partir de tout type de charge hydrocarbonée. L'équation générale correspondant à la réaction de la première étape (étape a)) devient alors pour une charge hydrocarbonée de type $C_aH_{2b}$ :

$$C_aH_{2b} + 2a\ H_2S \rightarrow a\ CS_2 + (2a+b)\ H_2$$

où a est un entier de préférence compris entre 1 et 30, bornes incluses, de préférence encore compris entre 1 et 20, bornes incluses, de préférence encore compris entre 1 et 10, bornes incluses, et b représente un entier compris entre a/2 et 2(a + 1), bornes incluses, avec la restriction que lorsque a représente 1 alors b représente 2.

Ainsi, et à titre d'exemple illustratif, lorsque la charge hydrocarbonée est le propane ($C_3H_8$, a = 3 et b = 4) par exemple, on obtiendrait potentiellement 3 $CS_2$ et 10 $H_2$.

Ainsi, la charge hydrocarbonée mise à réagir avec le sulfure d'hydrogène ($H_2S$) dans l'étape a) peut être de tout type connu de l'homme du métier et généralement est une charge hydrocarbonée sous forme gazeuse, liquide ou solide, de préférence sous forme gazeuse ou liquide, de préférence encore sous forme gazeuse, et comprenant au moins un hydrocarbure ayant une chaîne hydrocarbonée sous forme linéaire ou ramifiée, cyclique, saturée ou insaturée.

De préférence encore, la charge hydrocarbonée comprend au moins un alcane, préférentiellement au moins le méthane ($CH_4$), l'éthane, le propane ou le butane, et de manière tout à fait préférée le méthane. Avantageusement encore, la charge hydrocarbonée est pure, c'est-à-dire qu'elle contient un seul composé, par exemple, un alcane, et préférentiellement le méthane ($CH_4$), l'éthane, le propane ou le butane, et de manière tout à fait préférée le méthane.

La charge hydrocarbonée définie ci-dessus peut provenir de nombreuses sources, toutes connues de l'homme du métier, qu'elles soient naturelles, artificielles ou synthétique, par exemple à partir de sources naturelles, mais aussi par synthèse directe, par métathèse, et autres. Des exemples de sources de charge hydrocarbonée utilisables dans le procédé de la présente invention comprennent de manière illustrative et non limitative la biomasse, le pétrole, le charbon, la houille, les schistes bitumineux, les sables bitumineux, et autres.

Selon un aspect tout particulièrement préféré, la charge hydrocarbonée mise en oeuvre à l'étape a) est choisie parmi le gaz naturel, le gaz de schiste, le pétrole de schiste. De préférence, les sources de charges hydrocarbonées sont choisies parmi le gaz naturel, le gaz de schiste, et le biogaz.

D'autres exemples de sources de charges hydrocarbonées qui peuvent avantageusement être utilisées dans le cadre de la présente invention comprennent les naphtas, les produits de distillation du pétrole brut, les coupes pétrolières, de préférence démétallisées, désoxygénées et/ou désazotées, les produits de décomposition, et en particulier de la méthanisation, naturelle ou industrielle de la biomasse.

Néanmoins, dans le cadre de la présente invention, on préfère utiliser, en tant que charge hydrocarbonée de départ, le méthane, principalement en raison de raisons économiques, notamment avec les développements récents dans l'exploitation du gaz de schiste.

Le méthane, utilisé comme charge hydrocarbonée de départ, peut être mis en oeuvre avec un ou plusieurs autres gaz, autres que des charges hydrocarbonées telles que précédemment décrites mais pour des raisons évidentes de purification ultérieures et de facilité de conduite du procédé (risque d'accumulation avec d'éventuels recyclages) on préférera utiliser que des mélanges de charges hydrocarbonées ou du méthane pur.

Dans le cas de l'utilisation de méthane pur, mais aussi lorsque la charge hydrocarbonée de départ est autre que du méthane seul, il n'y a pas de réelles contraintes au niveau du ratio molaire $H_2S/CH_4$, ou $H_2S$/charge hydrocarbonée, que l'on peut utiliser dans l'étape a), puisque que l'excès d'$H_2S$ est avantageusement recyclé à l'issue de l'étape b). Si le sulfure d'hydrogène est mis en oeuvre en quantité sous-stoechiométrique, l'impact se verra au niveau de la conversion du méthane, ou de la charge hydrocarbonée respectivement, et de la production d'hydrogène.

On peut également envisager de ne pas mettre d'$H_2S$ à la première étape et générer « in situ » l'$H_2S$ nécessaire en faisant réagir la charge hydrocarbonée avec le soufre selon la réaction (3) définie plus haut. Le ratio molaire $H_2S$/charge hydrocarbonée peut donc être égal à 0 (si du soufre est présent) et aller jusqu'à environ 100, de préférence le ratio molaire est compris entre 0,5 et 10 et de préférence encore entre 1 et 3, ces plages de valeurs s'entendant bornes incluses. Ces valeurs sont particulièrement adaptées lorsque la charge hydrocarbonée de départ est le méthane ou comprend du méthane.

La charge hydrocarbonée et le sulfure d'hydrogène sont avantageusement apportés en continu ou discontinu dans le ou les réacteurs dans lequel le procédé selon l'invention est mis en oeuvre, en particulier selon que le procédé est mis en oeuvre en continu ou en « batch ». Avantageusement, la charge hydrocarbonée et le sulfure d'hydrogène sont sous forme liquide ou solide ou gazeuse, de préférence sous forme gazeuse.

Selon un mode de réalisation, l'étape a) est mise en oeuvre en l'absence de soufre, ce qui correspond au procédé de l'invention selon le schéma ($\alpha$). Selon un autre mode de réalisation, l'étape a) est mise en oeuvre en présence de soufre, ce qui correspond au procédé de l'invention selon le schéma ($\beta$). Dans ce mode de réalisation, le soufre est sous forme liquide, solide ou gazeuse, de préférence, sous forme liquide ou gazeuse.

Le procédé selon la présente invention offre en outre le grand avantage de générer, quel que soit le mode de réalisation ($\alpha$) ou ($\beta$), un excédent d'hydrogène qui peut être séparé du milieu réactionnel et valorisé. Une valorisation particulièrement intéressante pour l'hydrogène formé est une combustion avec de l'oxygène pour fournir de l'énergie thermique nécessaire aux différentes étapes du procédé de l'invention, notamment pour le chauffage de la réaction de l'étape a) qui demande des températures élevées pour des performances acceptables industriellement.

Ainsi et selon un autre aspect, l'invention concerne un procédé de synthèse de disulfure de diméthyle tel que défini précédemment, dans lequel l'hydrogène co-produit est totalement ou en partie transformé en énergie thermique, par exemple par combustion (dans l'oxygène ou dans l'air, par exemple), cette énergie thermique pouvant être utilisée pour le chauffage de la ou des réactions de l'étape a), de l'étape b) et/ou de l'étape d), de préférence de l'étape a).

Selon un mode de réalisation, l'étape a) est réalisée en présence d'un catalyseur. Dans ce mode de réalisation, ledit catalyseur comprend avantageusement un métal de transition choisi parmi les éléments des colonnes 6 à 11 de la classification périodique des éléments (groupes VIB, VIIB, VIIIB), de préférence parmi les éléments des colonnes 6, 9 et 10, et de préférence encore le catalyseur comprend un ou plusieurs métaux de transition choisis parmi le platine, le rhodium, le chrome et le palladium. De préférence encore, le catalyseur comprend un ou plusieurs métaux de transition choisis parmi le platine, le rhodium, le chrome ou le palladium, de manière tout à fait préféré, le catalyseur comprend du platine.

Ainsi, le catalyseur de l'étape a) comprend un métal ou des métaux, ceux-ci pouvant être en mélange, et celuici (ou ceux-ci) pouvant être sous forme métallique, mais aussi sous forme oxydé(s), sulfuré(s). Lorsque le catalyseur se présente sous la forme d'oxyde métallique, une étape de sulfuration peut être avantageusement réalisée selon les méthodes connues de l'homme du métier.

De préférence, le catalyseur utilisé à l'étape a) est un catalyseur supporté, le support étant choisi de préférence choisi parmi alumine, silice, zéolithes, charbons actifs, oxyde de titane, oxyde de zirconium, argiles, hydrotalcite, hydroxyapatite, magnésie, et autres. Le catalyseur peut-être favorablement utilisé en lit fixe, fluide, circulant ou bouillonnant. De préférence, le catalyseur est utilisé en lit fixe. Selon un autre mode de réalisation, l'étape a) est réalisée en l'absence de catalyseur.

La température réactionnelle dans l'étape a) est avantageusement comprise entre 500°C et 1300°C, de préférence entre 700°C et 1100°C, de préférence encore entre 800°C et 1000°C. Pour des raisons de conversion pour la limite basse et de tenue des matériaux pour la limite haute, on préfère une gamme de température comprise entre 700°C

et 1100°C, de préférence comprise entre 800°C et 1000°C.

La réaction de l'étape a) peut être indifféremment réalisée à pression atmosphérique, sous pression, voire sous dépression, l'homme du métier sachant adapter les conditions de pression réactionnelles selon la nature des réactifs mis en oeuvre, les températures de réaction choisies, les vitesses de circulation des flux et les taux de conversion et les rendements visés.

De manière générale, l'étape a) peut être réalisée sous une pression comprise entre 50 mbars et 100 bars (soit entre $5.10^3$ et $1.10^7$ Pa), de préférence encore entre la pression atmosphérique et 50 bars (soit $5.10^6$ Pa), et avantageusement entre la pression atmosphérique et 15 bars, (soit $15.10^5$ Pa).

La durée de la réaction de l'étape a) peut varier dans de grandes proportions, selon notamment la nature et la quantité de chacun des réactifs, la nature et la quantité de catalyseur utilisé, la température et la pression choisies. De manière générale, la durée de réaction de l'étape a) peut varier entre quelques secondes à quelques minutes.

Lorsque du soufre est présent pour la mise en oeuvre de la réaction de l'étape a), le ratio molaire soufre/$CH_4$ est préférentiellement compris entre 0 et 4, bornes exclues ou plus généralement le ratio molaire soufre/charge hydrocarbonée est préférentiellement compris entre 0 et (2a+b), bornes exclues, où a et b sont tels que définis précédemment.

Un ratio molaire soufre/$CH_4$ égal ou supérieur à 4, compte tenu de la réaction (3), pourrait entraîner la conversion totale du méthane en $CS_2$ et $H_2S$, ce qui n'est pas souhaitable pour l'étape b) du procédé qui nécessite de l'hydrogène. Selon un aspect préféré de la présente invention, le ratio soufre/$CH_4$ est ainsi compris entre 0 et 4, bornes exclues, de préférence entre 0 et 2,5, bornes exclues et de préférence encore entre 0 et 1,5, bornes exclues.

Comme indiqué précédemment, le procédé selon l'invention permet de s'affranchir d'une étape de purification entre les étapes a) et b). En effet, lors de la mise en oeuvre de l'étape b), l'hydrogène ($H_2$) et le disulfure de carbone ($CS_2$) obtenus à l'étape a) réagissent directement ensemble pour former du sulfure d'hydrogène ($H_2S$) et du méthylmercaptan ($CH_3SH$), et éventuellement de l'hydrogène ($H_2$). Ainsi, les ratios respectifs des réactifs mis en oeuvre dans l'étape b) sont directement dépendants des ratios des produits obtenus à l'issue de l'étape a).

La conduite de la réaction de l'étape b) est connue de l'homme du métier et décrite par exemple dans la demande internationale WO2010/046607. Cette réaction est ainsi connue pour conduire à une conversion du $CS_2$ de 100% pour une sélectivité en méthylmercaptan de 100%, si l'hydrogène est à la stoechiométrie ou en excès. La conséquence est que le méthylmercaptan produit dans cette étape b) est très facile à séparer du milieu réactionnel car celui-ci ne contient que du méthylmercaptan, de l'$H_2S$, de l'hydrogène s'il était en excès et éventuellement la charge hydrocarbonée qui pourrait être en excès dans l'étape a), pour avoir une conversion totale du soufre. Il convient de noter que la charge hydrocarbonée en excès, après passage de façon inerte dans l'étape b) et séparation du méthylmercaptan formé, peut être recyclée à l'étape a) avec l'$H_2S$.

Selon un mode réalisation, l'étape b) peut être réalisée en présence d'un catalyseur. Dans un mode de réalisation préféré, un catalyseur est utilisé pour l'hydrogénation du disulfure de carbone en méthylmercaptan. Le catalyseur qui peut être utilisé peut être de tout type connu de l'homme du métier comme catalyseur d'hydrogénation. Avantageusement, le catalyseur utilisé pour l'étape b) du procédé selon la présente invention peut être choisi parmi ceux décrits dans la demande internationale WO2010/046607, dans laquelle ledit catalyseur d'hydrogénation comprend au moins un métal dopé par au moins un hydroxyde ou oxyde d'alcalin ou d'alcalino-terreux.

Le métal présent dans le catalyseur de l'invention peut être tout métal du groupe 6 et/ou 8 du tableau périodique de la classification des éléments (IUPAC), et de préférence choisi dans le groupe comprenant nickel (Ni), cobalt (Co), palladium (Pd), rhodium (Rh), platine (Pt), molybdène (Mo), tungstène (W), chrome (Cr), fer (Fe) et les combinaisons de deux ou plusieurs d'entre eux, de préférence les combinaisons de deux de ces métaux, et en particulier Co/Mo, Ni/Mo, Ni/W, W/Mo, les combinaisons de nickel et de molybdène étant tout particulièrement préférées.

Le métal ou les métaux présent(s) dans le catalyseur de l'invention peuvent également se présenter directement sous la forme de sulfures métalliques. Ces sulfures métalliques peuvent aussi être obtenus à partir des oxydes correspondants selon toute méthode connue de l'homme du métier.

Le catalyseur de l'invention est avantageusement supporté, de manière conventionnelle, sur tout type de support généralement utilisé dans ce domaine, et par exemple sur un support choisi parmi alumine, silice, dioxyde de titane ($TiO_2$), zéolites, charbon, zircone, magnésie (MgO), les argiles, les hydrotalcites et autres, ainsi que les mélanges de deux ou plusieurs d'entre eux.

Comme pour le catalyseur utilisé à l'étape a), le catalyseur mis en oeuvre est favorablement utilisé en litfixe, fluide, circulant ou bouillonnant. De préférence, le catalyseur est en lit fixe.

La quantité de catalyseur utilisée dans l'étape a) et la quantité de catalyseur utilisé dans l'étape b) dépendent de la quantité de méthylmercaptan que l'on veut obtenir. Ainsi, les quantités de catalyseur(s) mises en oeuvre dans les étapes a) et b) sont ajustées dans le but d'obtenir une productivité en méthylmercaptan allant de 0,1 $kg.h^{-1}$ à 20 $kg.h^{-1}$ par litre de catalyseur. Dans cette configuration, le procédé selon la présente invention s'est montré particulièrement intéressant en termes de rentabilité économique et industrielle. Selon un autre mode de réalisation, l'étape b) est réalisée sans catalyseur.

La température réactionnelle de l'étape b) est généralement plus faible que celle utilisée dans l'étape a), et est

communément comprise entre 100°C et 400°C et de préférence entre 200°C et 300°C, gamme de température dans laquelle on observe la sélectivité maximale en méthylmercaptan, et ce, pour une conversion optimale.

Comme pour l'étape a), l'étape b) peut être réalisée sous toute pression, de préférence comprise entre 50 mbars et 100 bars (soit entre $5.10^3$ Pa et $1.10^7$ Pa) de préférence encore entre la pression atmosphérique et 50 bars (soit $5.10^6$ Pa) et avantageusement entre la pression atmosphérique et 15 bars (soit $15.10^5$ Pa).

La durée de l'hydrogénation varie selon la nature et la quantité de chacun des réactifs, et la nature et la quantité de catalyseur utilisé. Par exemple, la réaction varie entre quelques secondes et quelques minutes.

Comme indiqué précédemment l'étape d) de réaction du soufre sur le méthylmercaptan produisant du disulfure de diméthyle et du sulfure d'hydrogène peut être réalisée selon toute méthode connue de l'homme du métier, le sulfure d'hydrogène formé pouvant être recyclé à l'étape a) ou à l'étape c). Les conditions opératoires pour la mise en oeuvre de l'étape d) sont par exemple décrites dans EP0976726.

Les étapes a), b) et d) peuvent être mises en oeuvre dans tout type de réacteur aptes à recevoir des réactions à haute température, par exemple réacteurs en alliage, de type Hastelloy, Incoloy, et autres.

Selon un mode de réalisation préféré, les étapes a), b) et d) sont chacune mise en oeuvre dans un réacteur distinct. Selon un autre mode de réalisation, les étapes a), b) et d) sont réalisées successivement dans le même réacteur.

Comme indiqué précédemment, le procédé selon l'invention comprend éventuellement, mais de préférence une étape c) de recyclage du sulfure d'hydrogène formé à l'issue de l'étape b) et qui est réintroduit dans la charge de départ pour réalisation de l'étape a). Cette étape c) de recyclage du sulfure d'hydrogène formé présente l'avantage qu'il est ainsi possible d'éviter la synthèse *ex situ* de sulfure d'hydrogène.

De même, le procédé selon l'invention comprend éventuellement, mais de préférence une étape e) de recyclage du sulfure d'hydrogène formé à l'issue de l'étape d) et qui est réintroduit dans la charge de départ pour réalisation de l'étape a) et/ou réintroduit dans la recycle de sulfure d'hydrogène à l'étape c).

Le sulfure d'hydrogène peut ainsi être recyclé après séparation du milieu réactionnel de l'étape b) et/ou de l'étape d), selon toute méthode connue de l'homme du métier, et par exemple par distillation, de préférence sous pression, par cryogénie, par séparation membranaire, et autres.

L'étape f) de récupération de disulfure de diméthyle peut être réalisée selon toute méthode connue en soi et par exemple par dégazage des composés plus volatils, tels que l'hydrogène et le sulfure d'hydrogène. La charge hydrocarbonée éventuellement non convertie, ainsi que le disulfure de carbone et/ou le soufre éventuellement non converti(s) sont séparés du méthylmercaptan par distillation.

L'ensemble du milieu réactionnel de l'étape d) restant (duquel a été retiré le DMDS) peut être avantageusement réintroduit/recyclé dans l'étape a) du procédé. Ce mode de réalisation présente l'avantage de recycler également la charge hydrocarbonée de départ, ce qui permet d'améliorer substantiellement le rendement en production de DMDS par rapport à la charge hydrocarbonée introduite au départ. Le procédé est ainsi optimisé du fait que chaque atome de carbone présent dans la charge hydrocarbonée de départ est converti en une demi-mole de méthylmercaptan.

Ainsi et selon une variante, le procédé selon l'invention comprend, outre le recyclage du sulfure d'hydrogène, le recyclage des composés résiduels, c'est-à-dire non réagis, c'est-à-dire disulfure de carbone, éventuellement hydrogène, éventuellement de la charge hydrocarbonée, éventuellement du soufre et éventuellement des impuretés. De manière générale, le recyclage est réalisé selon des techniques bien connues de l'homme du métier.

Il a en outre pu être observé que, lorsque du coke est formé au cours de la mise en oeuvre du procédé selon l'invention, celui-ci réagit avec le sulfure d'hydrogène (et éventuellement le soufre présent) pour former de l'hydrogène et du disulfure de carbone. Le procédé selon l'invention présente donc le très grand avantage d'être opéré comme un système parfaitement autonome, et sans émission gênante de sulfure d'hydrogène, sans formation gênante de coke dans le réacteur malgré la présence de charge hydrocarbonée à haute température.

La présente invention offre ainsi un procédé industriel de préparation de DMDS totalement autonome, de rendement élevé, plus respectueuse de l'environnement et plus économique que les méthodes connues dans l'art antérieur.

Dans une variante du procédé de l'invention, lorsque les sous-produits ne sont pas recyclés, ou lorsque seul le sulfure d'hydrogène est recyclé, il est possible de valoriser lesdits sous-produits que sont le sulfure d'hydrogène, l'hydrogène et éventuellement le disulfure de carbone. Une utilisation particulièrement intéressante de l'hydrogène formé au cours du procédé de l'invention est sa mise en oeuvre avec du soufre liquide pour former du sulfure d'hydrogène qui peut être ainsi utilisé dans le procédé de l'invention, comme cela a déjà été indiqué plus haut.

Grâce aux avantages précités du procédé selon la présente invention, il est possible d'atteindre une productivité en méthylmercaptan élevée, en général de l'ordre de 0,1 kg à 20 kg de méthylmercaptan par heure et par litre de catalyseur de l'étape b), méthylmercaptan qui est ensuite converti en DMDS (étape d)) après recyclage éventuel du sulfure d'hydrogène formé (étape c).

Selon un mode de réalisation préféré, le sulfure d'hydrogène formé à l'étape c) est recyclé dans l'étape a). Selon un autre mode de réalisation préféré, le procédé selon l'invention comprend une étape e) dans laquelle le sulfure d'hydrogène de l'étape d) est recyclé dans l'étape a). Selon encore un autre mode de réalisation préféré le sulfure

d'hydrogène de l'étape c) et le sulfure d'hydrogène de l'étape e) sont recyclés dans l'étape a). Selon encore un autre mode de réalisation préféré le sulfure d'hydrogène de l'étape e) est recyclé dans l'étape c).

Il a ainsi été démontré que le procédé de synthèse de disulfure de diméthyle selon la présente invention offre de très nombreux avantages par rapport aux procédés connus de l'art antérieur et en particulier celui de s'affranchir de l'utilisation de méthanol, entraînant ainsi des coûts de production plus faibles.

La présente invention est maintenant illustrée au moyen des exemples qui suivent, qui ne présentent aucun caractère limitatif et qui ne peuvent être par conséquent compris comme susceptible de restreindre la portée de l'invention telle que revendiquée.

EXEMPLES

Pour chacun des exemples, les produits réactionnels et les produits n'ayant pas réagi sont vaporisés et analysés par une chromatographie en phase gazeuse avec une colonne capillaire munie d'un détecteur (microGC, colonne tamis/PPU en série avec une colonne PoraPLOT de Agilent Technologies, détecteur $\mu$TCD).

Dans les exemples *infra,* les taux de conversion et sélectivité sont déterminés comme suit :

- Taux de conversion molaire du $CH_4$ ($\%C_{CH4}$) :

$$\%C_{CH4} = [(n_{0CH4} - n_{CH4\ résiduel}) / n_{0CH4}] * 100$$

avec $n_{0CH4}$ étant le nombre de moles initial de $CH_4$ et $n_{CH4\ résiduel}$ étant le nombre de moles de $CH_4$ n'ayant pas réagi.

- Taux de conversion molaire de $CS_2$ ($\%C_{CS2}$) :

$$\%C_{CS2} = [(n_{0CS2} - n_{CS2\ résiduel}) / n_{0CS2}] * 100$$

avec $n_{0CS2}$ étant le nombre de moles initial de $CS_2$ et $n_{CS2\ résiduel}$ étant le nombre de moles de $CS_2$ n'ayant pas réagi.

- Sélectivité molaire en $CH_3SH$ ($\%SCH_3SH$) :

$$\%S_{CH3SH} = [n_{CH3SH} / (n_{0CS2} - n_{CS2\ résiduel})] * 100$$

avec $n_{CH3SH}$ étant le nombre de moles de $CH_3SH$ produites au cours du procédé selon l'invention.

- Sélectivité molaire en $CS_2$:

$$\%S_{CS2} = [n_{CS2} / (n_{0CH4} - n_{CH4\ résiduel})] * 100$$

avec $n_{CS2}$ étant le nombre de moles de $CS_2$ produites au cours du procédé selon l'invention.

**Exemple 1** :

Un réacteur en Incoloy® 800 HT contenant 12 grammes de catalyseur contenant 0,5% en poids de platine sur alumine commercialisé par la société STREM est placé dans un four. Le catalyseur est intercalé entre deux couches de carborundum.

On alimente le réacteur avec 20 $NL.h^{-1}$ (soit 893 $mmol.h^{-1}$) de sulfure d'hydrogène ($H_2S$) et 10 $NL.h^{-1}$ (soit 446 $mmol.h^{-1}$) de méthane ($CH_4$). Ces deux gaz sont indépendamment préchauffés à 500°C avant leur entrée dans le réacteur. Le réacteur est porté à l'aide du four à une température de 900°C et la pression à la sortie du réacteur est régulée à 3 bars absolus. Le débit des gaz de sortie, ramené dans les conditions normales de température et de pression, c'est-à-dire 0°C et 1 atmosphère (101325 Pa), est de 37,5 $NL.h^{-1}$.

L'analyse par chromatographie en phase gazeuse des gaz de sortie indique que 4 gaz sont présents : $CH_4$ et $H_2S$ non convertis ainsi que $CS_2$ et $H_2$ qui ont été produits avec un ratio molaire $H_2/CS_2$ de 4. Dans ces conditions, la conversion molaire du $CH_4$ est de 32%, avec une sélectivité en $CS_2$ de 100%.

Ces gaz de sortie après refroidissement à une température régulée de 250°C sont introduits dans un deuxième réacteur contenant 50 mL de catalyseur NiMo/alumine (HR448, commercialisé par la société Axens), dopé avec 11,6% de $K_2O$ (selon la préparation « Cata 3 » décrite dans la demande WO2010/046607). La pression est de 3 bars (0,3 MPa) absolus dans le four, à 250°C. L'analyse par chromatographie en phase gazeuse des gaz de sortie nous montre que

le $CS_2$ a été totalement converti (100%) avec une sélectivité de 100% en méthylmercaptan, c'est-à-dire que chaque molécule de disulfure de carbone a été convertie en méthylmercaptan suivant la réaction (4). Le milieu réactionnel comprend également du sulfure d'hydrogène, de l'hydrogène, ainsi que du méthane non réagi. L'ensemble de ces composés peut être recyclé dans l'étape a).

**Exemple 2** :

L'exemple 1 a été répété en ajoutant cette fois 5,7 $g.h^{-1}$ de soufre (soit 178 $mmol.h^{-1}$) aux 10 $NL.h^{-1}$ de méthane (soit 446 $mmol.h^{-1}$) et en réduisant les 20 $NL.h^{-1}$ d'$H_2S$ à 10 $NL.h^{-1}$ (446 $mmol.h^{-1}$). Le soufre est introduit sous forme liquide à 130°C avec les autres réactifs, en haut du réacteur, dont la température interne est maintenue à une température de 900°C et la pression interne à 3 bars ($3.10^5$ Pa) absolus. Le débit des gaz de sortie ramené dans les conditions normales de température et de pression est de 28 $NL.h^{-1}$.

L'analyse par chromatographie en phase gazeuse des gaz de sortie indique la composition molaire suivante : $CH_4$ : 21% (soit 262 $mmol.h^{-1}$), $H_2S$ : 22% (soit 275 $mmol.h^{-1}$), $CS_2$ : 14% (soit 175 $mmol.h^{-1}$) et $H_2$ : 43% (soit 537 $mmol.h^{-1}$).

Le bilan matière réalisé avec ces analyses indique que le soufre a été converti à 100%, que le méthane a été converti à 39% en disulfure de carbone ($CS_2$), et que le $CS_2$ et l'hydrogène ($H_2$) ont été produits avec un ratio molaire $H_2/CS_2$ de 3,07.

De la même façon que dans l'exemple 1, les gaz de sortie après refroidissement à une température régulée de 250°C sont introduits dans le deuxième réacteur contenant 50 mL de catalyseur NiMo/alumine (HR448 d'Axens) dopé avec 11,6% de $K_2O$. La pression est de 3 bars absolus.

L'analyse par chromatographie en phase gazeuse des gaz de sortie indique que le $CS_2$ a été converti à 100% avec une sélectivité de 100% en méthylmercaptan (soit 175 $mmol.h^{-1}$). En outre, la quantité d'$H_2S$ récupérée à l'issue de cette deuxième étape correspond, aux erreurs de mesure près, à la quantité nécessaire à la première étape (soit environ 450 $mmol.h^{-1}$). Le procédé selon l'invention est un système autonome qui permet avantageusement le recyclage des composés résiduels vers l'étape 1), par exemple $H_2S$. Le $CS_2$ et l'hydrogène n'ont pas été quantifiables.

Cet exemple démontre qu'il est tout à fait possible d'envisager un procédé de synthèse de méthylmercaptan dans lequel la totalité de l'$H_2S$ produit pourrait être recyclé, et n'aurait pas besoin d'être synthétisé pour les besoins dudit procédé de synthèse de méthylmercaptan.

Les exemples suivants illustrent encore le procédé de la présente invention comme indiqué dans l'exemple 1 ci-dessus, mais où la première étape a été reproduite avec différents catalyseurs.

**Exemple 3 :**

Le catalyseur de la première étape de l'exemple 1 a été remplacé par 30 mL d'un catalyseur contenant 2% en poids de palladium sur alumine (Société Engelhard). La réaction a ensuite été effectuée à 700°C, 800°C et 900°C. Les résultats sont regroupés dans le Tableau 1.

**Exemple 4** :

Le catalyseur de la première étape de l'exemple 1 a été remplacé par 60 cm de fil de platine de 0,4 mm de diamètre. La réaction a ensuite été effectuée à 900°C. Les résultats sont regroupés dans le Tableau 1.

**Exemple 5 :**

Le catalyseur de la première étape de l'exemple 1 a été remplacé par 20 toiles superposées (épaisseur d'une toile = 0,152 mm, volume des 20 toiles = 0,611 mL) faites de platine et de rhodium (Société Umicore). La réaction a ensuite été effectuée à 900°C, 1000°C et 1100°C. Les résultats sont regroupés dans le Tableau 1.

**Exemple 6 :**

Le catalyseur de la première étape de l'exemple 1 a été remplacé par 30 mL d'un catalyseur contenant 19% en poids d'oxyde de chrome ($CrO_2O_3$) sur alumine (T2777, commercialisé par la société Süd-Chemie). Le catalyseur a subi un traitement préalable de sulfuration avec un flux d'$H_2S$ (20 $NL.h^{-1}$) pendant 4 heures à 900°C, de façon à convertir le $Cr_2O_3$ en $Cr_2S_3$ et à éviter la formation de produits oxygénés pendant la réaction principale entre le méthane et l'$H_2S$. Ces produits oxygénés pourraient être gênants dans les étapes de récupération ultérieure du méthylmercaptan. La réaction a ensuite été effectuée à 900°C. Les résultats sont regroupés dans le Tableau 1 suivant :

**-- Tableau 1 --**

| *N° exemple* | *température (°C)* | *conversion $CH_4$%* | *sélectivité $CS_2$%* |
|---|---|---|---|
| 3 | 700 | 9 | 100 |
| 3 | 800 | 12 | 100 |
| 3 | 900 | 18 | 100 |
| 4 | 900 | 11 | 100 |
| 5 | 900 | 30 | 100 |
| 5 | 1000 | 57 | 100 |
| 5 | 1100 | 85 | 100 |
| 6 | 900 | 28 | 100 |

**Exemple 7 :**

Le milieu réactionnel contenant le méthylmercaptan (MeSH) obtenu à l'exemple 2 est introduit dans un réacteur contenant de la résine Amberlyst A21 sèche pour produire, en présence de soufre, du DMDS comme décrit dans EP976726. Du soufre liquide est introduit dans ce réacteur. La pression opératoire est maintenue à 5,5 bars (0,55 MPa) relatifs et la température à 40°C. Le mélange réactionnel en sortie de réacteur a la composition pondérale suivante hors MeSH en excès et hors $H_2S$ : DMDS 85%, polysulfures de diméthyle (DMPS) 15%.

Ce mélange réactionnel est alors envoyé dans un dégazeur pour être traité. Après traitement, le mélange débarrassé de l'$H_2S$ est envoyé dans un réacteur finisseur qui contient une charge de résine Amberlyst A21 sèche. La pression et la température dans le réacteur finisseur sont identiques à celles du réacteur principal.

À la sortie du réacteur finisseur, le mélange a la composition pondérale suivante hors H2S et hors MeSH en excès : DMDS 98,5%, DMPS 1,5%. Le mélange est alors introduit dans un dégazeur pour élimination de $H_2S$ formé dans le réacteur lors de la rétrogradation des polysulfures de diméthyle par le MeSH pour donner du DMDS.

Après le dégazage ci-dessus, le mélange est introduit dans une colonne de distillation où les impuretés volatiles telles que le méthylmercaptan (MeSH) et le sulfure de diméthyle (DMS) sont éliminés en tête de colonne. Le DMDS recueilli en fond de colonne a la composition pondérale suivante :

- DMDS : 99,7%
- DMPS : 3000 ppm
- MeSH : < 100 ppm
- DMS : < 50 ppm

**Revendications**

1. Procédé de préparation de disulfure de diméthyle, en batch ou en continu, de préférence en continu, ledit procédé comprenant au moins les étapes suivantes :

   a) réaction d'au moins une charge hydrocarbonée en présence de sulfure d'hydrogène ($H_2S$) et optionnellement de soufre (S) pour former du disulfure de carbone ($CS_2$) et de l'hydrogène ($H_2$),
   b) réaction d'hydrogénation dudit disulfure de carbone ($CS_2$) en présence dudit hydrogène ($H_2$) obtenus à l'étape a), pour former du méthylmercaptan ($CH_3SH$), du sulfure d'hydrogène($H_2S$) et éventuellement de l'hydrogène ($H_2$),
   c) éventuellement, mais de préférence, recyclage dudit sulfure d'hydrogène($H_2S$) formé à l'étape b) vers l'étape a),
   d) réaction du méthylmercaptan formé à l'étape b) avec du soufre pour former du disulfure de diméthyle et du sulfure d'hydrogène,
   e) éventuellement recyclage dans l'étape a) du sulfure d'hydrogène formé à l'étape d), et
   f) récupération du disulfure de diméthyle.

2. Procédé selon la revendication 1, dans lequel la charge hydrocarbonée est une charge hydrocarbonée sous forme

gazeuse, liquide ou solide, de préférence sous forme gazeuse ou liquide, de préférence encore sous forme gazeuse, et comprenant au moins un hydrocarbure ayant une chaîne hydrocarbonée sous forme linéaire ou ramifiée, cyclique, saturée ou insaturée.

**3.** Procédé selon la revendication 1 ou la revendication 2, dans lequel la charge hydrocarbonée comprend au moins un alcane, préférentiellement au moins le méthane ($CH_4$), l'éthane, le propane ou le butane.

**4.** Procédé selon l'une quelconque des revendications précédentes, dans lequel la charge hydrocarbonée est le méthane.

**5.** Procédé selon l'une quelconque des revendications précédentes, dans lequel le sulfure d'hydrogène formé dans l'étape b) est recyclé dans l'étape a).

**6.** Procédé selon l'une quelconque des revendications précédentes, dans lequel l'hydrogène éventuellement formé à l'étape b) peut être mis en réaction avec du soufre pour former du sulfure d'hydrogène.

**7.** Procédé selon l'une quelconque des revendications précédentes, dans lequel le sulfure d'hydrogène formé dans l'étape d) est recyclé dans l'étape a).

**8.** Procédé selon l'une quelconque des revendications précédentes, dans lequel le ratio molaire $H_2S$/charge hydrocarbonée est compris entre 0,5 et 10 et de préférence encore entre 1 et 3, bornes incluses.

**9.** Procédé selon l'une quelconque des revendications précédentes, dans lequel la température réactionnelle dans l'étape a) est avantageusement comprise entre 500°C et 1300°C, de préférence entre 700°C et 1100°C, de préférence encore entre 800°C et 1000°C.

**10.** Procédé selon l'une quelconque des revendications précédentes, dans lequel la température réactionnelle dans l'étape b) est comprise entre 100°C et 400°C et de préférence entre 200°C et 300°C.

**11.** Procédé selon l'une quelconque des revendications précédentes, dans lequel l'hydrogène co-produit est totalement ou en partie transformé en énergie thermique, cette énergie thermique pouvant être utilisée pour le chauffage de la ou des réactions de l'étape a), de l'étape b) et/ou de l'étape d).

## Patentansprüche

**1.** Verfahren zur diskontinuierlichen oder kontinuierlichen, vorzugsweise kontinuierlichen, Herstellung von Dimethyldisulfid, wobei das Verfahren mindestens die folgenden Schritte umfasst:

a) Reaktion von mindestens einer Kohlenwasserstoffcharge in Gegenwart von Schwefelwasserstoff ($H_2S$) und gegebenenfalls Schwefel (S) zur Bildung von Kohlendisulfid ($CS_2$) und Wasserstoff ($H_2$),
b) Hydrierungsreaktion des Kohlendisulfids ($CS_2$) in Gegenwart des Wasserstoffs ($H_2$), die in Schritt a) erhalten wurden, zur Bildung von Methylmercaptan ($CH_3SH$), Schwefelwasserstoff ($H_2S$) und gegebenenfalls Wasserstoff ($H_2$),
c) gegebenenfalls, aber vorzugsweise, Rezyklierung des in Schritt b) gebildeten Schwefelwasserstoffs ($H_2S$) zu Schritt a),
d) Reaktion des in Schritt b) gebildeten Methylmercaptans mit Schwefel zur Bildung von Dimethyldisulfid und Schwefelwasserstoff,
e) gegebenenfalls Rezyklierung des in Schritt b) gebildeten Schwefelwasserstoffs in Schritt a) und
f) Gewinnung des Dimethyldisulfids.

**2.** Verfahren nach Anspruch 1, bei dem es sich bei der Kohlenwasserstoffcharge um eine Kohlenwasserstoffcharge in gasförmiger, flüssiger oder fester Form, vorzugsweise in gasförmiger oder flüssiger Form, noch weiter bevorzugt in gasförmiger Form, handelt und die Kohlenwasserstoffcharge mindestens einen Kohlenwasserstoff mit einer Kohlenwasserstoffkette in cyclischer, gesättigter oder ungesättigter linearer oder verzweigter Form umfasst.

**3.** Verfahren nach Anspruch 1 oder Anspruch 2, bei dem die Kohlenwasserstoffcharge mindestens ein Alkan, vorzugsweise mindestens Methan ($CH_4$), Ethan, Propan oder Butan, umfasst.

**4.** Verfahren nach einem der vorhergehenden Ansprüche, bei dem es sich bei der Kohlenwasserstoffcharge um Methan handelt.

**5.** Verfahren nach einem der vorhergehenden Ansprüche, bei dem der in Schritt b) gebildete Schwefelwasserstoff in Schritt a) rezykliert wird.

**6.** Verfahren nach einem der vorhergehenden Ansprüche, bei dem der gegebenenfalls in Schritt b) gebildete Wasserstoff mit Schwefel zu Schwefelwasserstoff umgesetzt werden kann.

**7.** Verfahren nach einem der vorhergehenden Ansprüche, bei dem der in Schritt d) gebildete Schwefelwasserstoff in Schritt a) rezykliert wird.

**8.** Verfahren nach einem der vorhergehenden Ansprüche, bei dem das $H_2S$/Kohlenwasserstoffcharge-Molverhältnis zwischen 0,5 und 10 und weiter bevorzugt zwischen 1 und 3 einschließlich der Grenzen liegt.

**9.** Verfahren nach einem der vorhergehenden Ansprüche, bei dem die Reaktionstemperatur in Schritt a) vorteilhafterweise zwischen 500 °C und 1300 °C, vorzugsweise zwischen 700 °C und 1100 °C, noch weiter bevorzugt zwischen 800 °C und 1000 °C, liegt.

**10.** Verfahren nach einem der vorhergehenden Ansprüche, bei dem die Reaktionstemperatur in Schritt b) zwischen 100 °C und 400 °C und vorzugsweise zwischen 200 °C und 300 °C liegt.

**11.** Verfahren nach einem der vorhergehenden Ansprüche, bei dem der als Nebenprodukt anfallende Wasserstoff ganz oder teilweise in Wärmeenergie umgewandelt wird, wobei diese Wärmeenergie zum Erwärmen der Reaktion bzw. Reaktionen von Schritt a), von Schritt b) und/oder von Schritt d) verwendet werden kann.

## Claims

**1.** Process for preparing dimethyl disulphide, batchwise or continuously, preferably continuously, said process comprising at least the following steps:

a) reaction of at least one hydrocarbon charge in the presence of hydrogen sulphide ($H_2S$) and optionally of sulphur (S) to form carbon disulphide ($CS_2$) and hydrogen ($H_2$),
b) hydrogenation reaction of said carbon disulphide ($CS_2$) in the presence of said hydrogen ($H_2$), both obtained in step a), to form methyl mercaptan ($CH_3SH$), hydrogen sulphide ($H_2S$) and optionally hydrogen ($H_2$),
c) optionally, but preferably, recycling of said hydrogen sulphide ($H_2S$) formed in step b) to step a),
d) reaction of the methyl mercaptan formed in step b) with sulphur to form dimethyl disulphide and hydrogen sulphide,
e) optionally recycling into step a) of the hydrogen sulphide formed in step d), and
f) recovery of the dimethyl disulphide.

**2.** Process according to Claim 1, wherein the hydrocarbon charge is a hydrocarbon charge in gaseous, liquid or solid form, preferably in gaseous or liquid form, more preferably in gaseous form, and comprises at least one hydrocarbon having a hydrocarbon chain in saturated or unsaturated linear, branched or cyclic form.

**3.** Process according to Claim 1 or Claim 2, wherein the hydrocarbon charge comprises at least one alkane, preferably at least methane ($CH_4$), ethane, propane or butane.

**4.** Process according to any one of the preceding claims, wherein the hydrocarbon charge is methane.

**5.** Process according to any one of the preceding claims, wherein the hydrogen sulphide formed in step b) is recycled into step a).

**6.** Process according to any one of the preceding claims, wherein the hydrogen optionally formed in step b) may be reacted with sulphur to form hydrogen sulphide.

**7.** Process according to any one of the preceding claims, wherein the hydrogen sulphide formed in step d) is recycled

into step a).

**8.** Process according to any one of the preceding claims, wherein the molar $H_2S$/hydrocarbon charge ratio is between 0.5 and 10 and more preferably between 1 and 3, endpoints included.

**9.** Process according to any one of the preceding claims, wherein the reaction temperature in step a) is advantageously between 500°C and 1300°C, preferably between 700°C and 1100°C, more preferably between 800°C and 1000°C.

**10.** Process according to any one of the preceding claims, wherein the reaction temperature in step b) is between 100°C and 400°C and preferably between 200°C and 300°C.

**11.** Process according to any one of the preceding claims, wherein the co-produced hydrogen is wholly or partly converted to thermal energy, it being possible for this thermal energy to be used for the heating of the reaction or reactions of step a), of step b) and/or of step d).

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 337837 A
- EP 446109 A
- WO 2004096760 A
- WO 2006015668 A
- WO 2007028708 A
- WO 2008118925 A
- WO 2013092129 A
- US 2007213564 A
- US 2008293974 A
- US 2010094059 A
- US 2010286448 A
- WO 2010046607 A
- US 3880933 A
- WO 200196290 A
- WO 2004022482 A
- EP 0976726 A
- EP 976726 A